# EUROPEAN PATENT APPLICATION

(11) **EP 1 990 398 A1**
(43) Date of publication of application: **12.11.2008**
(21) Application number: 08075478.1
(22) Date of filing: 09.05.2008
(51) Int. Cl.: C10L 1/19, C07C 67/03, C11B 1/02, C11B 3/06, C11C 3/10, C10L 1/02

(54) **Process for preparing fatty acid esters from pre-treated glyceride oils**

(30) Priority: 11.05.2007 GB 0709100
(71) Applicant: N.V. De Smet Engineering S.A., 1935 Zaventem (BE)
(72) Inventor: Kellens, Marc, 2812 Mechelen-Muizen (BE); Adami, Icilio, 20049 Concorezzo (Milano) (IT); Soragna, Francesco, 00128 Roma (IT); de Greyt, Wim, 9112 Sinaai (BE)
(74) Representative: Bird, Ariane

(57) **Abstract**

This invention provides a_process for forming fatty acid esters of C₁₋₄ alkyl alcohols by alkaline transesterification of a glyceride oil, comprising the steps of:
(a) providing a glyceride oil with a free fatty acid content below 2 % more preferably below 0.5 weight % expressed as oleic acid;
(b) mixing said glyceride oil with at least a part of one or more alcoholic phases originating from the alkaline transesterification as referred to above;
(c) separating the mixture thus formed into a heavy, alcoholic phase and a light, pre-treated fatty phase; and
(d) transesterifying said pre-treated fatty phase with said C₁₋₄ alkyl alcohols.

The transesterified product can be used in biodiesel production.

## Description

### FIELD OF THE INVENTION

The invention relates to a process for pre-treating glyceride oils before said oils are subjected to a transesterification process with C₁₋₄ alkyl alcohols for the production of fatty acid esters suitable for inclusion into bio-diesel.

### BACKGROUND OF THE INVENTION

Biodiesel is synthesised from triglyceride oils by a process of transesterification with lower C₁₋₄ alkyl alcohols such as methanol. The triglyceride oils used for this purpose can be vegetable oils, such as soya bean oil, which is the predominant source in the US, rapeseed oil in Europe or palm oil in Malaysia. Non-edible vegetable oils such as *Jatropha curcas* are also being considered as raw material for biodiesel to ease the oil demand. The triglyceride oils can also be of animal origin such as for instance beef tallow.

Natural oils and fats often contain not insignificant amounts of free fatty acid (FFA). The free fatty acid content of crude oils depends to a high degree on their quality. In very general terms, the quality deteriorates as the FFA content rises. Good crude oils have FFA in the range of less than 5%, e.g. in the range of 0.5 - 3%. As described in WO 92/00268 these free acids react with a basic catalyst that has been added for transesterification purposes. The FFA interferes with the catalyst resulting in a part of the catalyst being neutralised and thus being prevented from being used for the tranesterification process. To solve this problem, it is known to neutralise or remove the FFA's, e.g. to refine the oil, before the transesterification process, otherwise one has to use a large amount of basic catalyst. It is known that transesterification can be carried out at atmospheric or slightly raised pressure with a slight excess of lower alcohols, at temperatures around the boiling point of such alcohols and also provided the oils and fats that are used have been treated previously by methods such as distillation, alkali extraction, acid catalysis esterification in order to reduce the FFA content to less than 0.5%.

Fully refined oils of good quality (e.g. refined, bleached, and deodorized products of high quality) have free fatty acid contents of less than 0.1%, i.e. well below the level at which FFA will interfere with a basic catalyst. A normal range of FFA for a refined oil of good quality is 0.01 - 0.05% FFA, in most cases related to oleic acid content.

For the transesterification, a basic catalyst such as sodium or potassium hydroxide or sodium methylate is generally used. As indicated above when the raw material contains free fatty acids, these acids will react with the catalyst under formation of soaps so that additional amounts of catalysts are required. Moreover, the soaps can complicate the phase separation between the fatty acid esters and the glycerol formed during the transesterification. Accordingly, the triglyceride oil used as the starting material is preferably a near-neutral oil with a free fatty acid content of less than 0.5 % by weight calculated as oleic acid, more preferably less than 0.1%, e.g. in the range 0.01-0.05%. This reduces the amount of catalyst required and facilitates phase separation.

Because slimy substances such as phosphatides also react with the transesterification catalyst and may cause phase separation problems, they should also be removed from the raw material. U.S. Patent No. 6,960,673 discloses a process wherein a crude oil or fat loaded with slimy substances is treated with a mixture used at 0.1 wt.% to 5 wt. % in relation to the oil or fat and containing an alcohol and concentrated acid, wherein said slimy substances swelled and being no longer oil-soluble or fat-soluble due to the treatment are subsequently separated. In this process, the treated oil or fat may be washed for separating said slimy substances and free fatty acids with an alkaline glycerol phase originating from an alkaline transesterification reaction of the above-mentioned type and, after said washing process, the glycerol phase loaded with soaps of the free fatty acids and slimy substances may be separated as heavy phase from the neutral oil.

When a near-neutral oil is mixed with methanol and catalyst - and this holds to some extent also for all C₁₋₄ alkyl alcohols -, a two-phase system results because methanol is only poorly soluble in triglyceride oil. However, the methanol that dissolves will react with the triglycerides under formation of fatty acid methyl esters and partial glycerides. The latter are miscible with triglycerides and methanol so that after the reaction has proceeded to a certain extent, a single phase results. This causes the reaction rate to increase and the partial glycerides to react with methanol under formation of further fatty acid methyl esters and glycerol. The decrease of the concentration of partial glycerides also decreases the solubility of glycerol so that gradually, the glycerol formed is concentrated in a separate phase.

Accordingly, the reaction mixture forms two separate phases: a fatty phase comprising mainly fatty acid methyl esters but also appreciable amounts of mono-, di- and triglycerides as well as some methanol and glycerol, and an alcoholic phase comprising mainly glycerol but also appreciable amounts of methanol, soaps and some partial glycerides. In industrial practice, these two phases are separated and each phase is processed further. Since biodiesel specifications *inter alia* stipulate a maximum content of partial glycerides, said fatty phase is therefore mixed with a further amount of methanol and transesterification catalyst which causes the partial glycerides present in said fatty phase to react under formation of further fatty acid methyl esters and glycerol. Ultimately, a fatty acid methyl ester product with a sufficiently low partial glyceride content is obtained and after washing to remove residual glycerol traces, this can be sold as biodiesel.

Said alcoholic phase is also treated. Before this treatment, it may be combined with other product streams containing glycerol. The first step involves an evaporative treatment to recover methanol, which can then be recycled.It also involves an acidulation to recover the fatty acid moieties of the soaps present in said phase as free fatty acids. The partial glycerides present in the alcoholic phase will also be concentrated in this fatty acid fraction. This acidulation also leads to the formation of inorganic salts that have to be removed by filtration before the glycerol can be concentrated by distillation to obtain pure glycerol for outside sale.

### OBJECT OF THE INVENTION

Accordingly, it is an object of the invention to provide a process and apparatus for pre-treating glyceride oils before said oils are subjected to a transesterification process with C₁₋₄ alkyl alcohols for the production of fatty acid esters suitable for inclusion into bio-diesel. An advantage of the present invention is that can overcome at least one of the various disadvantages and shortcomings of the prior art processes for pre-treating oils to be used in transesterification processes.

### SUMMARY OF THE INVENTION

It has surprisingly been found that the above object can be attained in a process for forming fatty acid esters of C₁₋₄ alkyl alcohols by alkaline transesterification of a glyceride oil, comprising the steps of:
a) providing a glyceride oil with a free fatty acid content below 2.0 weight % expressed as oleic acid;
b) mixing said glyceride oil with at least a part of one or more alcoholic phases originating from the alkaline transesterification as referred to above;
c) separating the mixture formed in step (b) into a heavy, alcoholic phase and a light, pre-treated fatty phase; and
d) transesterifying said pre-treated fatty phase with said C₁₋₄ alkyl alcohols.

It is an advantage of the present invention to decrease the amount of basic transesterification catalyst required for fatty acid methyl ester production. The use of refined oils and fats as a starting material (i.e. having 0.5 % by weight, more preferably less than 0.1%, e.g. 0.01-0.05% be weight FFA content) avoids interference of the FFA with the basic transesterification catalyst and hence can reduce the amount of basic transesterification catalyst that needs to be used.

It is also an advantage of the present invention to provide a transesterification process with reduced soap production.

It is also an advantage of the present invention to provide a transesterification process that is more robust than the prior art processes.

It is another advantage of the present invention to allow a more economical use to be made of the methanol required in the transesterification reaction.

### BRIEF DESCRIPTION OF THE DRAWINGS

FIGURE 1 is a flow diagram illustrating how crude triglyceride oil can be converted into fatty acid methyl esters and showing how the pre-treatment process according to the invention fits into said series of conversion steps.
FIGURE 2 is a flow diagram illustrating a specific embodiment of the invention, wherein the alcoholic phase formed in the interesterification step is re-used in the pre-treatment of glyceride oils.

### DEFINITION OF TERMS

As used herein, and unless otherwise stated, the term " transesterification " refers to the ester interchange reaction between an alcohol and a glyceride such as an oil or fat.

As used herein, and unless otherwise stated, the term " chemical refining "refers to the removal of free fatty acids by an alkali such as caustic soda.

As used herein, and unless otherwise stated, the term " degumming " refers to the removal of complex organo-phosphorus compounds such as phosphatides from oils and fats.

As used herein, and unless otherwise stated, the term " physical refining " refers to a process whereby free fatty acids are removed from a glyceride oil by a vacuum stripping process that commonly uses steam as the stripping medium.

As used herein the term "refined", as used in such phrases as refined oils or refined fats, means an oil or fat that has been refined by any suitable process such as distillation, alkali extraction, acid catalysis esterification in order to reduce the FFA content to less than 0.5 % by weight, more preferably less than 0.1%, e.g. 0.01-0.05% be weight such that the FFA does not interfere with the basic transesterification catalyst.

### DETAILED DESCRIPTION OF THE INVENTION

The pre-treatment process according to the invention can be applied to a wide range of raw materials destined to be converted into fatty acid esters or biodiesel by a transesterification process. In order to save on transesterification catalyst, this raw material preferably has a free fatty acid content of less than 0.5 wt% calculated as oleic acid is preferred, more preferably less than 0.1%, e.g. 0.01-0.05% be weight. If the free fatty acid content is considered to be too high, it should be lowered by conventional means. These can be a chemical refining process employing an alkali such as sodium hydroxide which neutralisation is optionally preceded by a degumming operation, or a physical refining process preferably preceded by a gum removal process such as a dry degumming process, an acid degumming or an acid refining process.

In the prior art processes, this near-neutral oil is mixed with anhydrous methanol and a transesterification catalyst after which the reaction mixture is allowed to settle to enable the two phases formed during the reaction to separate. The lighter fatty phase is then mixed with a further amount of methanol and catalyst to convert residual glycerides to fatty acid methyl esters and glycerol. The heavy alcoholic phase is processed to recuperate its constituents.

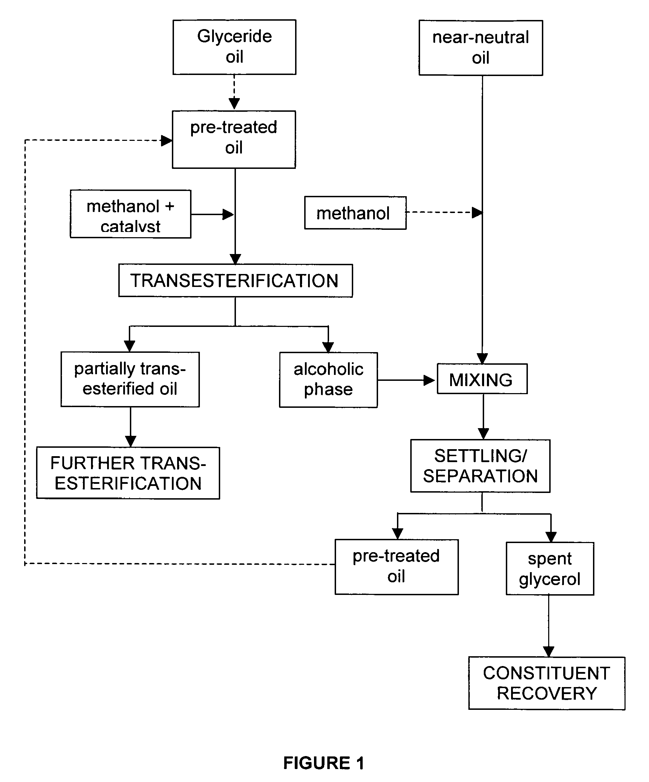

In the process according to the invention, this heavy alcoholic phase is not processed immediately but it is allowed to react first with glyceride oil. During this reaction, free fatty acids present in the glyceride oil are converted to soaps by the alkalinity still present in the heavy, alcoholic phase. In addition, and this is most surprising indeed, the heavy alcoholic phase also exhibits some residual catalytic activity. This means that if some methanol is mixed with the glyceride oil and the heavy alcoholic phase as indicated by the dotted arrow in FIG. 1, this causes some fatty acid methyl esters to be formed. Consequently, the pre-treatment process according to the invention may also induce some preliminary transesterification.

This preliminary transesterification diminishes the catalyst requirement in the subsequent main transesterification reaction. Moreover, the pre-treatment has also neutralised some free fatty acids present in the glyceride oil, so that these are no longer able to inactivate the basic catalyst; this also advantageously results into a significant catalyst saving.

The amount of heavy, alcoholic phase to be mixed with the glyceride oil is not a critical parameter of this invention. In industrial practice, the amount should preferably be equivalent to the amount that is liberated during the first transesterification step but the invention is in no way limited to this equivalency feature. In fact, glycerol-rich heavy phases resulting from subsequent transesterification steps can also be used to pre-treat the glyceride oil without departing from the spirit of the invention.

The amount of methanol to be mixed with the glyceride oil and the heavy, alcoholic phase originating from an alkaline transesterification is not a critical parameter of this invention and can vary within the range of 0-30 mol% of the fatty acid moieties present in the glyceride oil.

In the process according to the invention, the mixing intensity has been found to be an important process parameter of the mixing step (b) and although the process according to the invention is not limited to this type of mixer, the use of a mechanical mixer is preferred.

For practical reasons, the temperature of the pre-treatment process according to the invention is preferably around the atmospheric boiling point of methanol (64.6 °C). A high temperature increases reaction rate constants and may thus speed up the pre-treatment process. On the other hand, the temperature is preferably kept below 90°C so that the still modest pressure inside the equipment does not require expensive constructional measures to be taken.

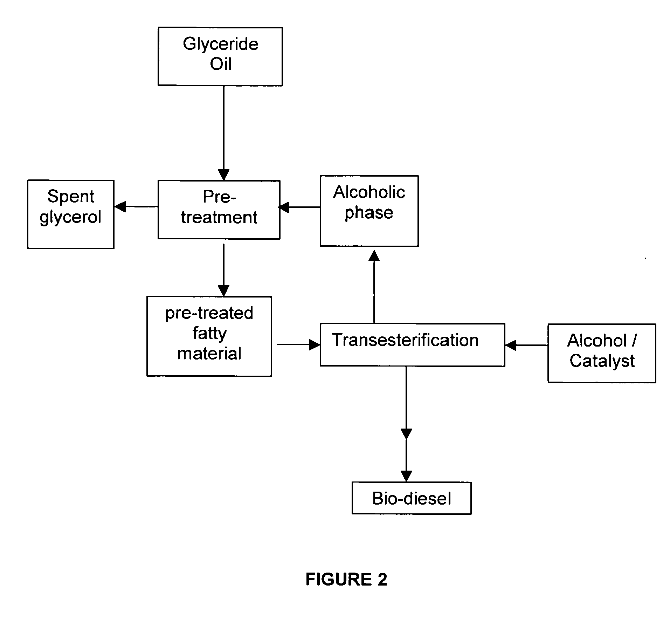

In the specific embodiment shown in Figure 2, a glyceride oil with a free fatty acid content below 0.5 weight % expressed as oleic acid, more preferably below 0.1%, e.g. 0.01-0.05%, is pre-treated, by mixing said glyceride oil with an alcoholic phase originating from a previous alkaline transesterification. The resulting mixture is separated into a heavy phase referred in Figure 2 as "spent glycerol", and a light, pre-treated fatty phase that is subjected to a transesterification step, using a C₁₋₄ alkyl alcohol and an alkaline catalyst. The mixture obtained after said transesterification step is separated into a light phase, which is to be further processed towards bio-diesel, and a heavy alcoholic phase, which still shows substantial alkalinity. This last heavy phase is then re-used in the pre-treatment of a subsequent batch of glyceride oil.

## Claims

1. Process for forming fatty acid esters of C₁₋₄ alkyl alcohols by alkaline transesterification of a glyceride oil, comprising the steps of:
(a) providing a glyceride oil with a free fatty acid content below 2 weight % expressed as oleic acid;
(b) mixing said glyceride oil with at least a part of one or more alcoholic phases originating from the alkaline transesterification as referred to above ;
(c) separating the mixture thus formed into a heavy, alcoholic phase and a light, pre-treated fatty phase; and
(d) transesterifying said pre-treated fatty phase with said C₁₋₄ alkyl alcohols.

2. Process according to claim 1, in which the free fatty acid content of said glyceride oil is below 0.5 weight % expressed as oleic acid.

3. Process according to claim 1, in which the free fatty acid content of said glyceride oil is below 0.1 weight % expressed as oleic acid.

4. Process according to claim 1, in which the free fatty acid content of said glyceride oil is between 0.01 and 0.05 weight % expressed as oleic acid.

5. Process according to any previous claim, in which the glyceride oil provided in step a) has been obtained by the chemical neutralisation of crude glyceride oil.

6. Process according to any of claims 1 to 5, in which the glyceride oil provided in step a) has been obtained by the physical refining of a crude or degummed glyceride oil.

7. Process according to any of claims 1 to 6, in which the amount of the heavy, alcoholic phase originating from an alkaline transesterification reaction that is added in step b), is substantially equivalent to the amount liberated by the alkaline transesterification reaction.

8. Process according to any of claims 1 to 7, in which anhydrous methanol is also added to the mixture resulting from step b).

9. Process according to claim 8, in which the amount of said anhydrous methanol is less than 30 mol % of the fatty acid moieties present in the glyceride oil provided in step a).

10. Process according to any of claims 1 to 9, in which a mechanical mixer is used in step b).

11. Process according to any of claims 1 to 10, in which the temperature of the reaction mixture arrived at in step b) is within the range of 40°C to 90°C.

12. Process according to any previous claim, wherein said fatty acid esters of C₁₋₄ alkyl alcohols resulting from step b) are further processed to bio-diesel fuel.

13. Process according to any of claims 1 to 11, wherein said fatty acid esters of C₁₋₄ alkyl alcohols resulting from step b) are included into bio-diesel fuel.
